# EUROPEAN PATENT APPLICATION

(11) **EP 4 697 340 A1**
(43) Date of publication of application: **18.02.2026**
(21) Application number: 25178145.6
(22) Date of filing: 22.05.2025
(51) Int. Cl.: G16H 15/00, G06F 40/30, G16H 50/30, G16H 50/70

(54) **METHOD AND SYSTEM FOR CLASSIFICATION OF PRE-ANESTHETIC PHYSICAL STATUS OF PATIENTS**

(30) Priority: 16.08.2024 KR 20240109745; 07.03.2025 KR 20250029977
(71) Applicant: Seoul National University Hospital, Seoul 03080 (KR)
(72) Inventor: LEE, Hyeonhoon, 02597 Seoul (KR); LEE, Hyung-Chul, 05504 Seoul (KR); LEE, Jipyeong, 02097 Seoul (KR)
(74) Representative: V.O.

(57) **Abstract**

The present application relates to a method and a system for automatically generating medical summary information and predicting pre-anesthetic physical status (ASA-PS) class of patients by analyzing various clinical data of an electronic medical record with a natural language processing technology. The present application extracts various clinical data from an electronic medical record database, such as surgical information, hospitalization initial diagnosis, nursing initial diagnosis, hospitalization progress, vital signs, test results, and clinical observation records of the patients, and then automatically generates medical summary information of the patients using an artificial intelligence-based natural language processing system. The generated medical summary information is used as an input of a medical classification model to classify the pre-anesthesia physical status of the patients, and visualizes and provides a prediction basis of the medical classification model.

## Description

### BACKGROUND

### 1. Field

The present application relates to a system and a method for generating medical summary information (premedi-summary) based on clinical data provided in an electronic medical record (EMR) using a natural language processing (NLP) model, thereby automatically predicting an American Society of Anesthesiologists Physical Status (ASA-PS), and providing interpretability of prediction results.

This research was conducted by Seoul National University Hospital under the support of the Ministry of Science and ICT's Individual Basic Research Program (Development and Validation of Machine Learning-Based Multiple Outcome Prediction Models in Critically Ill Patients Using Heart Rate Variability, Project ID: 2710001309) and the Ministry of Health and Welfare's Research-Oriented Hospital Development Program (Development of Ultra-Large Time-Series Multimodal AI Agents and Clinical Validation through Multi-Country Monitoring Platforms, Project ID: 2460001701).

### 2. Description of the Related Art

The classification of pre-anesthesia physical status is a very important process for evaluating the patient's systemic condition before surgery and predicting the risk that may occur during surgery. The American Society of Anesthesiologists Physical Status (ASA-PS) is a fundamental assessment tool widely used to evaluate co-morbidities in patients and predict intraoperative mortality and morbidity.

ASA-PS is widely used in anesthesia guidelines for sedation and pain control, ambulatory surgery, pre-procedure evaluations, and the like, and has an important influence on medical insurance billing and reimbursement. In particular, according to the guidelines of the Korean Health Insurance Review and Assessment Service, 50% additional anesthesia fee is applied to patients with ASA-PS class 3 or higher, so that accurate ASA-PS classification directly affects the financial operation of the hospital.

However, the current ASA-PS classification system has an issue of poor consistency due to differences in subjective judgment among healthcare professionals. Although the 2014 ASA tried to improve this problem by providing specific examples for each class, there are still inconsistencies among healthcare professionals in various clinical situations. In particular, there is a large difference in opinions between healthcare professionals in distinguishing ASA-PS classes 2 and 3, which may be an important issue in patient management and medical fee calculation.

Conventional technologies for predicting patient status through electronic medical record (EMR) analysis have been mainly based on structured data. Systems have been developed that analyze standardized data, such as diagnostic codes, test results, vital signs, to predict a patient status, or to support decision-making of healthcare professionals. In particular, advances in machine learning and artificial intelligence technologies have steadily enhanced the accuracy of such predictions.

However, in order for a clinician to evaluate an anesthesia patient before surgery, it is necessary to check various clinical data of 10 or more types, such as a hospitalization initial diagnosis, nursing initial diagnosis, hospitalization progress, vital signs of a hospital room, and perioperative test results, and manually prepare a summary of the patient status based on this, which takes a lot of time, and there is an issue in that the consistency of the summary is poor for each rater. In order to automatically predict the pre-anesthetic physical status (ASA-PS) class from the unstructured data present in the electronic medical record, there is a need for a technology for comprehensively analyzing and summarizing the dispersed clinical information.

In recent years, various advanced language models have emerged in the field of natural language processing (NLP), and the processing capability of unstructured medical text data has been greatly enhanced. Models are being developed that demonstrate superior performance in ASA-PS classification, particularly through learning specific to the medical field.

In addition, advances in large language model (LLM) and multi-agent collaboration network have made it possible to comprehensively analyze various clinical data and automatically generate medical summary information. Utilizing these technologies may automate patient status summaries previously written manually by clinicians to increase efficiency and ensure consistency.

Therefore, there is a need to develop a system that may automatically generate medical summary information by analyzing various clinical data of the electronic medical record utilizing these state-of-the-art natural language processing technologies, automatically predict ASA-PS based thereon, and provide a clear description of a prediction result. This is expected to minimize variability due to the subjective judgment of healthcare professionals and enable more objective and consistent ASA-PS classification.

### Prior Art Documents

### Patent Document

(Patent Document 1) KR 10-2024-0032196 A1 (2024.3.12)

### SUMMARY

It is a main object of the present application to provide a system and a method for automatically generating medical summary information by analyzing various clinical data of an electronic medical record, automatically classifying pre-anesthesia physical status (ASA-PS) class of the patients through a natural language processing model based on the information, and providing an interpretation of a prediction result thereof.

More specifically, a first object of the present application is to provide a system capable of extracting various clinical data such as hospitalization initial diagnosis, nursing initial diagnosis, hospitalization progress, vital signs of a hospital room, and perioperative test results from an electronic medical record, and based on the data, automatically generating medical summary information of the patients by utilizing large language model and multi-agent collaboration network. In particular, it is intended to enhance the quality of text data through accurate translation of medical terminology in medical records in which Korean and English are used interchangeably and semantic correction according to context.

A second object of the present application is to develop a natural language processing model capable of processing long text, so as to analyze comprehensive information of status of the patients without omission.

A third object of the present application is to enhance classification accuracy between ASA-PS classes II and III. In particular, it is intended to minimize the misclassification rate in the classification of patients with ASA-PS class III or higher, for whom additional anesthesia fees are applied.

A fourth object of the present application is to provide an interpretability for a prediction result of a model. Various model explainability methods are utilized to visualize key medical terms and phrases that have influenced the prediction, thereby allowing the healthcare professionals to clearly understand the basis of the model's judgment.

A fifth object of the present application is to quantify and provide uncertainty of model prediction. The reliability of the prediction result may be evaluated by separately estimating the uncertainty due to the variability of the input data (aleatoric uncertainty) and the uncertainty due to a model parameter (epistemic uncertainty).

A sixth object of the present application is to evaluate the performance of a model through subgroup analysis according to a text length, and provide an optimal prediction result tailored to each situation.

Through solving these technical problems, the present application aims to support the decision-making of healthcare professionals in the process of pre-anesthetic patient evaluation, increase the objectivity and consistency of ASA-PS classification, and ultimately contribute to enhancement of patient safety and quality of medical service.

According to an aspect of the present application for solving the aforementioned technical challenges, there is provided a method for classification of pre-anesthetic physical status of patients using unstructured text data provided in an electronic medical record, the method including: extracting medical records from an electronic medical record database, the medical records including at least one or more of surgical information, hospitalization initial diagnosis, nursing initial diagnosis, hospitalization progress, vital signs, test results, and clinical observation records of the patients; generating medical summary information of patients from the extracted medical record using an artificial intelligence-based natural language processing system; classifying the pre-anesthetic physical status of the patients using the generated medical summary information as an input of a medical classification model; and visualizing and providing a predictive basis of the medical classification model.

In an embodiment of the present application, the medical summary information comprises a text in which Korean and English are used interchangeably, and generating the medical summary information of the patients further includes: performing an accurate translation of the medical terms with reference to a medical terminology dictionary before inputting into the medical classification model; and correcting a meaning of the medical terms according to the context and translating the text into English.

In an embodiment of the present application, generating the medical summary information of the patients includes: analyzing the extracted medical records to automatically identify the primary diagnosis and clinical condition of the patients; matching the relevant drug use history and the previous surgical history based on the diagnosis content of the patients; comparing and contrasting a plurality of test results with each other, and classifying the test results by diseases; and integrating the analysis, matching and classification results into a standardized form of medical summary information.

In an embodiment of the present application, the artificial intelligence-based natural language processing system includes at least one of large language model or multi-agent collaboration network.

In an embodiment of the present application, visualizing a predictive basis of the medical classification model includes: visualizing an impact of each portion of the input text on the prediction results using model explainability methods, wherein the contribution of key medical terms related to at least one or more of the patient's major disease, surgical history, current condition is highlight, and separately displaying the contribution of each input text to the prediction result by different visual elements, wherein a direction and a magnitude of the contribution is distinguishably visualized.

In an embodiment of the present application, the pre-anesthesia physical status of patients comprises an American Society of Anesthesiologists Physical Status (ASA-PS) class.

In an embodiment of the present application, the ASA-PS class is classified into one of classes I, II, III, IV-V, and the method further includes determining whether to apply additional anesthesia fees for the patients classified as ASA-PS class III or higher.

In an embodiment of the present application, for learning the medical classification model, the method includes: a training step using a first dataset; a tuning step using a second dataset; and a testing step using a third dataset, wherein the second dataset and the third dataset: comprise samples selected through a sampling method that takes into account each pre-anesthesia physical status class, and are configured such that no data from the same patient is overlapped between the second dataset and the third dataset.

In an embodiment of the present application, a plurality of board-certified anesthesiologists independently perform evaluation on the second dataset and the third dataset, and when the evaluation result is inconsistent, a final agreement is reached through additional board-certified anesthesiologist consultation to generate a reference label, wherein the evaluation is performed after removing a part explicitly mentioned in the ASA-PS classification information from the medical summary information.

In an embodiment of the present application, a plurality of predictions are performed to estimate an uncertainty for the prediction of the medical classification model, wherein the uncertainty is estimated by distinguishing between the uncertainty caused by variability inherent in the input data and the uncertainty caused by the model parameters.

In an embodiment of the present application, the method further includes: classifying the input text into subgroups according to the length of the text, and evaluating the performance of the medical classification model for each subgroup.

According to another aspect of the present application, there is provided a computer-readable recording medium having recorded thereon a program for executing the method for classification of pre-anesthetic physical status of patients in a computer.

According to another aspect of the present application, there is provided a system for classification of pre-anesthetic physical status of patients, the system comprising: an electronic medical record database; an extracting unit configured to extract medical records from an electronic medical record database, the medical records comprising at least one or more of surgical information, hospitalization initial diagnosis, nursing initial diagnosis, hospitalization progress, vital signs, test results, and clinical observation records of the patients; a summary generation unit configured to generate medical summary information of patients from the extracted medical record using the artificial intelligence-based natural language processing system; a medical classification model unit configured to classify the pre-anesthetic physical status of the patients using the generated medical summary information as an input of a medical classification model; and a visualization unit configured to visualize and provide a predictive basis of the medical classification model.

In an embodiment of the present application, the medical summary information comprises a text in which Korean and English are used interchangeably, the summary generation unit performs an accurate translation of the medical terms with reference to a medical terminology dictionary, and corrects a meaning of the medical terms according to a context.

In an embodiment of the present application, the summary generation unit may perform a function of analyzing the extracted medical records to automatically identify the primary diagnosis and clinical condition of the patients; a function of matching the relevant drug use history and the previous surgical history based on the diagnosis content of the patients; a function of comparing and contrasting a plurality of test results with each other, and classifying the test results by diseases; and a function of integrating the analysis, matching and classification results into a standardized form of medical summary information.

In an embodiment of the present application, the artificial intelligence-based natural language processing system comprises at least one of large language model or multi-agent collaboration network.

In an embodiment of the present application, the visualization unit visualizes an impact of each portion of the input text on the prediction results using model explainability methods, wherein the contribution of key medical terms related to at least one or more of the patient's major disease, surgical history, current condition is highlight, and separately display the contribution of each input text to the prediction result by different visual elements, wherein a direction and a magnitude of the contribution is distinguishably visualized.

In an embodiment of the present application, the pre-anesthesia physical status of patients comprises an American Society of Anesthesiologists Physical Status (ASA-PS) class, and further comprising an anesthesia fee calculation unit configured to determine whether to apply additional anesthesia fees for patients classified as ASA-PS class III or higher.

In an embodiment of the present application, the medical classification model unit comprises a transformer-based model capable of processing a long medical text, and provides high accuracy in pre-anesthesia physical status classification.

In an embodiment of the present application, the learning data of the medical classification model comprises samples selected through a sampling method that takes into account each pre-anesthesia physical status class, and the samples comprise reference labels generated through evaluation by a plurality of board-certified anesthesiologists.

The present application automatically generates medical summary information by analyzing various clinical data of the electronic medical record with a natural language processing technology and predicts pre-anesthesia physical status class (ASA-PS) of the patients, thereby minimizing variability due to subjective judgment of healthcare professionals and enhancing objectivity and consistency of the ASA-PS classification.

The present application automates the task of confirming and summarizing various clinical data such as hospitalization initial diagnosis, nursing initial diagnosis, hospitalization progress, vital signs of the hospital room, and perioperative test results by an anesthesiologist before surgery, thereby significantly shortening the preparation time and reducing the workload of the clinician.

The present application automatically generates a patient status summary through the artificial intelligence-based natural language processing system, so that deviation between raters may be minimized and standardized summary information may be provided to ensure consistency of clinical information.

The present application provides a system that may accurately process and analyze clinical data in which Korean and English are used interchangeably, thereby resolving linguistic barriers that occur in the domestic medical environment and enhancing the quality of text data.

The natural language processing model of the present application may process long text, so that comprehensive information of the patient status may be analyzed without omission, which may solve the issue of information loss due to token limitation of existing models.

The present application provides a clear interpretation of prediction results by utilizing various model explainability methods, so that healthcare professionals may understand and trust the judgment basis of the model, which may promote the introduction of the artificial intelligence model into the clinical field.

The present application minimizes the misclassification rate for patients with ASA-PS class III or higher, thereby increasing the accuracy of anesthetic addition application and contributing to the revenue management of the hospital.

The present application quantifies and provides the uncertainty of the model prediction, so that healthcare professionals may evaluate the reliability of the prediction result and reflect it in the clinical decision-making.

The present application provides an optimal prediction result according to each situation through subgroup analysis according to the text length, thereby increasing the usability in various clinical environments.

The present application ensures the quality of learning data of a model through a reference label generation process in which a plurality of board-certified anesthesiologists participate, which may enhance the reliability of model prediction.

By comprehensively analyzing various clinical data, the method of the present application complements missing or inconsistent information to enable more accurate patient status summary, which may contribute to enhancing patient safety and quality of care.

The system of the present application provides the following effects in the process of pre-anesthetic patient evaluation: (1) Increased efficiency: Since the data is automatically extracted and analyzed from the electronic medical record to generate a summary, the manual working time of the clinician may be greatly reduced. (2) Ensuring consistency: A standardized summary is provided by the AI algorithm to minimize various deviations between raters and provide consistent clinical information. (3) Enhanced accuracy: By comprehensively analyzing a multiple clinical data, missing or mismatched information may be complemented, enabling more accurate patient status summary.

As a result, the present application may enhance the efficiency and accuracy of the process of pre-anesthetic patient evaluation, support the decision-making of the healthcare professionals, and ultimately contribute to the enhancement of patient safety and the quality of medical service.

### BRIEF DESCRIPTION OF THE DRAWINGS

FIG. 1 is a flowchart of a method for classification of a pre-anesthesia physical status of patients according to an embodiment of the present application.
FIG. 2 is a block diagram of a system for classification of a pre-anesthesia physical status of patients according to an embodiment of the present application.
FIG. 3 is a detailed flowchart illustrating a training and explanation generation process of a medical classification model according to an embodiment of the present application.
FIG. 4 is a dataset configuration diagram for learning, tuning, and testing of a medical classification model according to an embodiment of the present application.
FIG. 5 is a table showing characteristics of a training set, a tuning set, and a test set according to an embodiment of the present application.
FIG. 6A to FIG. 6H each show a graph comparing the performance of the ClinicalBigBird model, which is the medical classification model of the present application, and other comparison targets (BioClinicalBERT, GPT-4, anesthesiology resident, board-certified anesthesiologist) for each ASA-PS class.
FIG. 7A to FIG. 7E are each a table comparing ASA-PS classification performance of natural language processing models, anesthesiology resident, board-certified anesthesiologist according to an embodiment of the present application.
FIG. 8A to FIG. 8C are each a table comparing ASA-PS classification performance of natural language processing models for each subgroup classified based on the length of medical summary information according to an embodiment of the present application.
FIG. 9 is a diagram illustrating a confusion matrix for ASA-PS classification of natural language processing models (ClinicalBigBird, BioClinicalBERT, GPT-4) and healthcare professionals (anesthesiology resident, board-certified anesthesiologist) according to an embodiment of the present application.
FIG. 10A to FIG. 10D are each a Shapley plot illustrating the impact of input text on each ASA-PS class classification of the ClinicalBigBird model according to an embodiment of the present application.

### DETAILED DESCRIPTION

Hereinafter, some embodiments of this application will be described in detail with reference to illustrative drawings. In adding reference numerals to the components in each figure, the same components may have the same numerals as far as possible, even if they are indicated in other figures. In addition, in describing the present embodiments, when it is determined that a specific description of a related known configuration or function may obscure the gist of the present technical idea, a detailed description thereof may be omitted.

### Definitions

Where "comprise," "have," "consist of," etc., referred to the present specification are used, other parts may be added unless "only" is used. When the components are expressed in the singular, it may include the case of including the plural unless there is a special explicit description.

In the description of the components of the present application, terms such as first, second, A, B, (a), and (b) may be used. Unless otherwise specified, these terms are only used to distinguish the components from other components, and the nature, sequence, order, number, and the like of the components are not limited by the terms.

In the present application, "natural language processing (NLP)" refers to a field of artificial intelligence that enables a computer to understand and process a human language, and refers to a technology for performing tasks such as semantic analysis, contextual understanding, and classification of text.

In the present application, an "electronic medical record (EMR)" refers to a document in which medical treatment information of a patient is stored in an electronic form in a medical institution, and includes a diagnosis, a prescription, a test result, a surgical record, and the like of the patient.

In the present application, "medical summary information (premedi-summary)" refers to summary information of a medical status of a patient automatically generated based on various clinical data of the patient for pre-anesthesia evaluation, and includes a current disease, a past medical history, a dosing history, test results, and the like of the patient.

In the present application, "American Society of Anesthesiologists Physical Status (ASA-PS)" refers to a system for classifying systemic conditions of the patients as defined by the American Society of Anesthesiologists, and is classified into classes I to VI.

In the present application, "token" refers to a minimum unit divided to process text, and may be a word, a number, a special character, or the like.

In the present application, "model explainability methods" is a method for describing prediction results of a machine learning model, and refers to a technology for quantifying and visualizing the influence of each input characteristic on prediction, including various techniques such as SHapley Additive exPlanations (SHAP).

In the present application, "uncertainty" is an indicator of reliability of model prediction, and includes uncertainty due to variability of input data (aleatoric uncertainty) and uncertainty due to model parameters (epistemic uncertainty).

In the present application, "large language model (LLM)" is an artificial neural network-based language model learned with large-scale data, and refers to a model capable of performing various natural language processing tasks such as text generation, analysis, and translation.

In the present application, "multi-agent collaboration network" is a system in which multiple artificial intelligence agents cooperate with each other to perform complex tasks, and each agent is responsible for a specific task and has a structure in which the agents interact with each other.

In the present application, terms such as "unit," "module," "apparatus," or "system" may refer to a combination of hardware as well as software driven by that hardware. For example, the hardware may be a data processing device including a CPU, GPU, or other processors, and the software may refer to running processes, objects, executable files, execution threads, programs, and the like.

### System Structure and Method

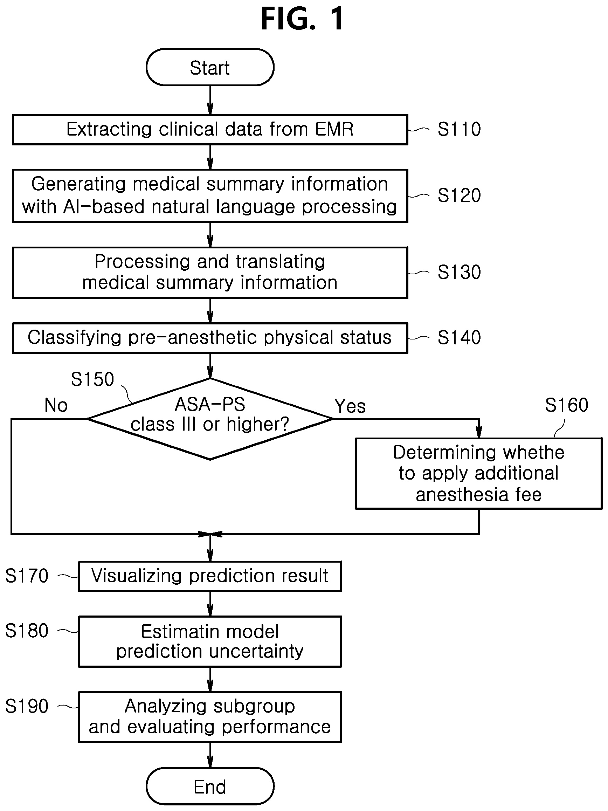

FIG. 1 is a flowchart of a method for classification of a pre-anesthesia physical status of patients according to an embodiment of the present application.

Referring to FIG. 1, in step S110, various clinical data of the patient (surgery information, hospitalization initial diagnosis, nursing initial diagnosis, hospitalization progress, vital signs of the hospital room, perioperative test, perioperative test image, perioperative test pathology, perioperative test function, clinical observation, and the like) are extracted from an electronic medical record (EMR) database. These clinical data include diagnostic information, past medical history, current medication, surgical history, and critical information related to anesthesia of the patients.

In step S120, medical summary information (premedi-summary) is generated using an artificial intelligence-based natural language processing system based on the extracted various clinical data. The medical summary information generation utilizes large language model and a multi-agent collaboration network. Each clinical data is analyzed to automatically identify main diagnosis and clinical status of the patients, match drug use history and surgical history, and classify various test results by disease to generate standardized form of medical summary information. The part where the ASA-PS classification information is explicitly mentioned in the generated medical summary information is removed, so that the medical classification model is not affected by the previous classification result.

In step S130, the generated medical summary information is processed to be used as an input of the medical classification model. In the case of a text in which Korean and English are used interchangeably, accurate translation of medical terms is performed with reference to the medical terminology dictionary, and the meaning of the medical terms is corrected according to the context. In particular, in the case of medical terminology, it is converted into standardized medical terms rather than simple translation to maintain consistency.

In step S140, the processed medical summary information is used as the input of the medical classification model to classify the pre-anesthesia physical status of the patient. The medical classification model used in the present application utilizes a transformer-based model with the ability to process long medical text. This model extracts and analyzes features important for pre-anesthesia physical status evaluation from medical summary information to predict ASA-PS class.

In step S150, it is determined whether the ASA-PS class predicted by the model is equal to or higher than III. ASA-PS class III or higher refers to a patient with severe systemic disease, which indicates a case where special attention is required for anesthesia management. This determination process is important for the establishment of a safe anesthesia management plan for the patient and the accuracy of medical fee calculation.

In step S160, it is determined whether to apply additional anesthesia fees for patients classified as ASA-PS grade III or higher. According to the guidelines of the Health Insurance Review and Assessment Service, 50% additional anesthesia fee may be applied to patients classified as ASA-PS grade III or higher. This process is an important step that directly affects the hospital's revenue management.

In step S170, visualization of the prediction result is performed. Various model explainability methods are used to visualize the impact of each portion of the input text on the prediction results. The contribution of major medical terms related to major disease, surgical history, current condition, and the like of the patients is highlighted and displayed, and the contribution of each input text is distinguishably displayed into different visual elements. Through this, the healthcare professionals may intuitively understand and verify the judgment basis of the model.

In step S180, the uncertainty of the model prediction is estimated. In this process, uncertainty due to variability of input data (aleatoric uncertainty) and uncertainty due to model parameters (epistemic uncertainty) are estimated separately. This makes it possible to evaluate the reliability of the prediction results and to identify cases that require further examination by the healthcare professionals. In the event of high uncertainty, the system provides a warning to the healthcare professionals, leading to a more careful review.

In step S190, subgroups are classified according to the length of the input text, and the performance of the model is evaluated for each subgroup. Through this, changes in the model's predictive performance according to text length can be analyzed, enabling the provision of optimal prediction results tailored to each situation. The results of the subgroup analysis are utilized for continuous improvement and optimization of the model.

The method of the present application demonstrates particularly high accuracy in distinguishing between ASA-PS grade II and grade III, and may greatly contribute to improving the accuracy of anesthesia management planning and medical fee calculation. By automatically analyzing the various clinical data of the electronic medical record to generate medical summary information and classify the pre-anesthetic physical status, it is possible to increase the work efficiency of the healthcare professionals and provide consistent evaluation results.

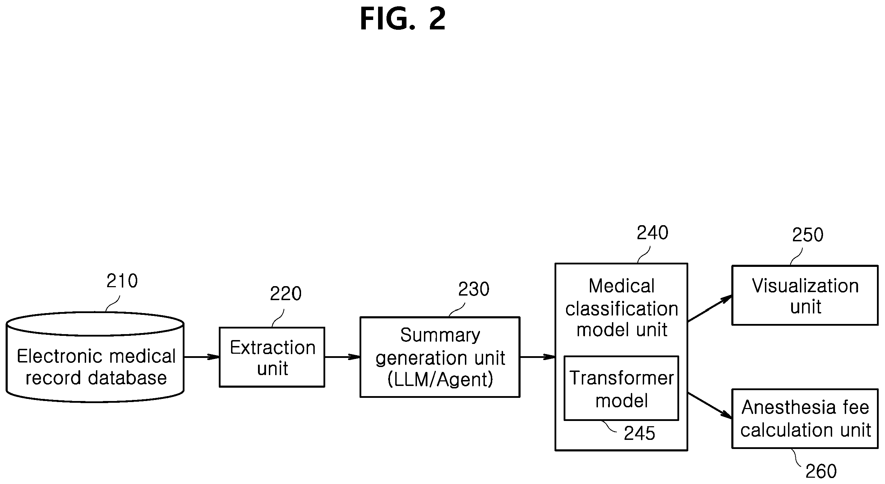

FIG. 2 is a block diagram of a system for classification of a pre-anesthesia physical status of patients according to an embodiment of the present application.

Referring to FIG. 2, a system of the present application includes an electronic medical record database 210, an extraction unit 220, a summary generation unit 230, a medical classification model unit 240, a visualization unit 250, and an anesthesia fee calculation unit 260.

The electronic medical record database 210 is a storage that stores medical treatment information of the patient in an electronic form, and includes various clinical data such as diagnostic information, prescription details, test results, and surgical records of the patient. In particular, various clinical data of 10 or more types, such as surgical information within the last 6 months, hospitalization initial diagnosis, nursing initial diagnosis, hospitalization progress, vital signs of the hospital room, perioperative test, perioperative test image, perioperative test pathology, perioperative test function, and clinical observation are stored.

The extraction unit 220 extracts various clinical data of the patient from the electronic medical record database 210. This clinical data contains important information necessary for pre-anesthesia patient evaluation and automatically extracts all information that had previously been manually checked by the clinician.

The summary generation unit 230 generates the patient's medical summary information (premedi-summary) using the artificial intelligence-based natural language processing system based on the extracted various clinical data. The summary generation unit 230 performs the following process by utilizing the large language model and the multi-agent collaboration network: (1) The clinical data is analyzed to automatically identify the major diagnosis and clinical status of the patient, (2) the related drug use history and previous surgical history are matched based on the diagnosis content of the patient, (3) various test results are compared and contrasted with each other to classify each disease, and (4) finally, standardized form of medical summary information is generated. The part where the ASA-PS classification information is explicitly mentioned in the generated medical summary information is removed to enable objective evaluation.

The medical classification model unit 240 receives the generated medical summary information as input and classifies the patient's pre-anesthesia physical status. **In** the case where Korean-English are used interchangeably, the medical summary information is used after translation and semantic correction with reference to the medical terminology dictionary. The medical classification model unit 240 includes a transformer model 245 having an encoder-decoder structure capable of processing long medical text. This model understands the context of the text and extracts key medical information to predict ASA-PS class.

The visualization unit 250 visualizes the prediction result of the medical classification model and the basis thereof. Various model explainability methods are used to visualize the impact of each portion of the input text on the prediction results, particularly highlighting the contribution of key medical terms. The visualization unit 250 helps the healthcare professionals to intuitively understand and verify the prediction result of the model.

The anesthesia fee calculation unit 260 determines whether to apply the anesthetic addition to the patient classified as ASA-PS class III or higher. According to the guidelines of the Health Insurance Review and Assessment Service, the 50% additional anesthesia fee may be applied to patients with ASA-PS III or higher, and the anesthesia fee calculation unit 260 plays a role of automating this fee calculation.

Each component of the system may be implemented as a software module executed by the processor of the data processing device, and may include a hardware accelerator as necessary. In particular, the medical classification model unit 240 may utilize an artificial intelligence-only processor such as a Graphics Processing Unit (GPU) or a Sensor Processing Unit (TPU) to enhance processing speed. Each component of the present system is configured to perform each step of the pre-anesthesia physical status classification method of the patient described in FIG. 1, and the extraction unit 220 is responsible for step S110, the summary generation unit 230 is responsible for step S120, the medical classification model unit 240 is responsible for steps S130 to S140, the anesthesia fee calculation unit 260 is responsible for steps S150 to S160, the visualization unit 250 is responsible for step S170, and the medical classification model units 240 and the visualization units 250 are jointly responsible for steps S180 to S190.

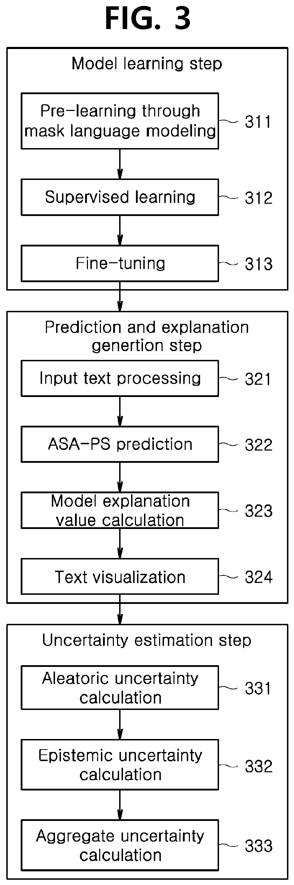

FIG. 3 is a detailed flowchart illustrating a training and explanation generation process of a medical classification model according to an embodiment of the present application.

Referring to FIG. 3, the present application consists of a model learning step, a prediction and explanation generation step, and an uncertainty estimation step.

The model learning step includes a pre-learning step 311 through mask language modeling, a supervised learning step 312, and a fine-tuning step 313. In the pre-learning step 311, large-scale medical text data is used to learn the model to understand the medical language. In the supervised learning step 312, the classification task is learned using the data with the ASA-PS label, and in the fine-tuning step 313, the model is optimized with high-quality data verified by board-certified anesthesiologists.

The prediction and explanation generation step consists of input text processing 321, ASA-PS prediction 322, model explanation value calculation 323, and text visualization 324 steps. In the input text processing step 321, the medical summary information is converted into a form that the model may process. The ASA-PS prediction step 322 predicts the ASA-PS class of patient based on the converted text. In the model explanation value calculation step 323, various explainability methods are utilized to calculate the influence of each input text on the prediction result. The explainability methods used in this case may include SHapley Additive exPlanations (SHAP), local interpretable model-agnostic explanations (LIME), attention visualization, integrated gradients, gradient-weighted class activation mapping (GradCAM), and the like. In particular, SHAP and attention visualization may be effectively utilized in a text-based model. The text visualization step 324 visualizes the calculated explanation values in an intuitively understandable form.

The uncertainty estimation step includes an aleatoric uncertainty calculation 331, an epistemic uncertainty calculation 332, and aggregate uncertainty calculation 333. The aleatoric uncertainty calculation step 331 calculates the uncertainty due to the inherent variability of the input data, and an epistemic uncertainty calculation step 332 calculates the uncertainty according to the model parameters. In the aggregate uncertainty calculation step 333, the two uncertainties are combined to evaluate the reliability of the final prediction.

With this detailed configuration of the present application, the model may go beyond simply predicting the ASA-PS class and provide the basis and reliability of the prediction together. The description generation process utilizing various model explainability methods helps the healthcare professionals to understand and verify the basis of the model's judgment, and uncertainty estimation may be utilized to evaluate the reliability of the prediction result. In particular, in the medical field, an accurate understanding of the model's decisions is critical, and therefore, it is important to appropriately combine various explanation techniques to provide explanations that may be trusted by the healthcare professionals.

### Experimental Example

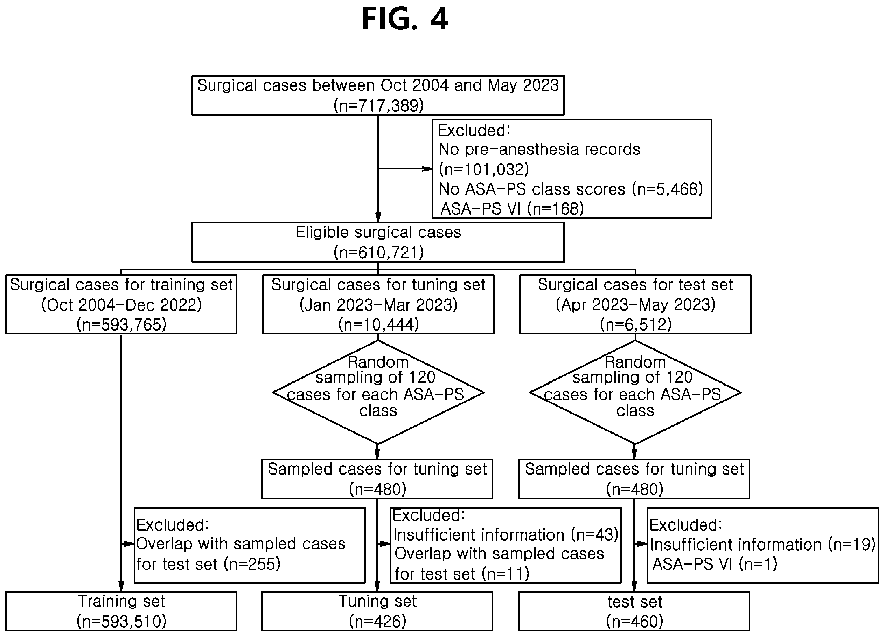

FIG. 4 is a dataset configuration diagram for learning, tuning, and testing of a natural language processing-based pre-anesthesia body status classification model according to an embodiment of the present application.

Referring to FIG. 4, a dataset was constructed for a total of 717,389 surgical cases collected at tertiary medical institutions from October 2004 to May 2023. Among them, 610,721 eligible surgical cases were finally selected, excluding cases without pre-anesthesia evaluation record (101,032 cases), cases without ASA-PS classification score (5,468 cases), and cases with ASA-PS class VI (168 cases).

Selected surgical cases were divided into three datasets by time period as follows:
- Cases from October 2004 to December 2022 (593,765 cases) were assigned to the training set.
- Cases from January to March 2023 (10,444 cases) were assigned to the tuning set.
- Cases from April to May 2023 (6,512 cases) were assigned to the test set.

For the tuning set and the test set, 120 cases were randomly extracted for each ASA-PS class to construct balanced datasets. During this process, cases with insufficient information and cases overlapped with other sets were excluded.

Finally:
- 593,510 cases in the training set,
- 426 cases in the tuning set, and
- 460 cases in the test set.

The tuning set and the test set were reference labels generated by a consensus panel composed of board-certified anesthesiologists, enabling objective evaluation of the performance of the model. In particular, the test set used the latest data temporally separated from the training set and tuning set to evaluate the generalization performance of the model.

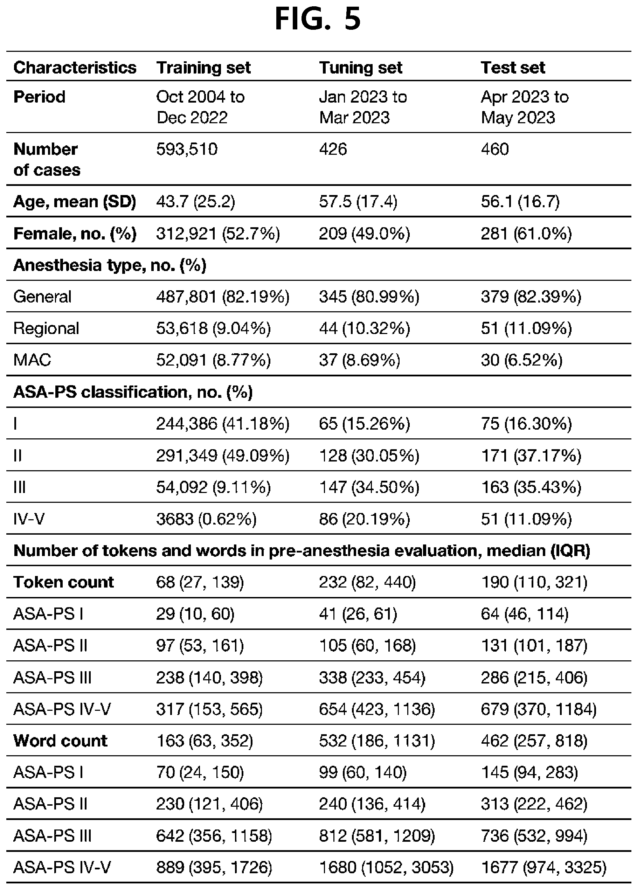

FIG. 5 is a table showing characteristics of a training set, a tuning set, and a test set according to an embodiment of the present application.

Referring to FIG. 5, the collection period of each dataset is from October 2004 to December 2022 for the training set, from January to March 2023 for the tuning set, and from April to May 2023 for the test set. The number of cases in each set is 593,510 for the training set, 426 for the tuning set, and 460 for the test set.

The mean age (standard deviation) of patients was 43.7 (25.2) years in the training set, 57.5 (17.4) years in the tuning set, and 56.1 (16.7) years in the test set. The proportion of female patients was 52.7% (312,921 patients) in the training set, 49.0% (209 patients) in the tuning set, and 61.0% (281 patients) in the test set.

By anesthesia type, general anesthesia was the most common with 82.19% (487,801 cases), 80.99% (345 cases), and 82.39% (379 cases), respectively, followed by regional anesthesia with 9.04% (53,618 cases), 10.32% (44 cases), and 11.09% (51 cases), and monitored anesthesia care (MAC) with 8.77% (52,091 cases), 8.69% (37 cases), and 6.52% (30 cases).

In the ASA-PS classification, ASA-PS I and II accounted for 90% of the total in the training set (I: 41.18%, II: 49.09%), but the tuning set and the test set were configured so that each ASA-PS class was relatively evenly distributed. This is to allow a fair evaluation even for the infrequent ASA-PS III or IV-V classes.

The length of medical summary information tended to increase with higher ASA-PS classes. The median number of tokens (IQR) increased from 29 (10, 60) in ASA-PS I cases to 317 (153, 565) in the ASA-PS IV-V cases, and the word count also increased from 70 (24, 150) in the ASA-PS I to 889 (395, 1726) in the ASA-PS IV-V, showing a corresponding upward trend. This indicates that the more severe the patient is, the more medical information is recorded.

The length of medical summary information was generally longer, especially in the tuning set and the test set, suggesting that recent medical records contain more detailed information than in the past. For example, for ASA-PS IV-V patients, the word count in the tuning set was 1680 (1052, 3053), and in the test set was 1677 (974, 3325), which is a significant increase from 889 (395, 1726) in the training set.

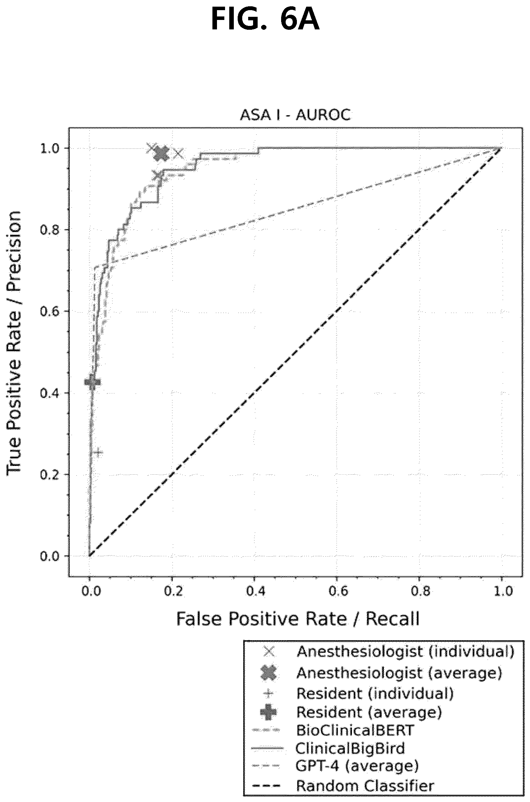

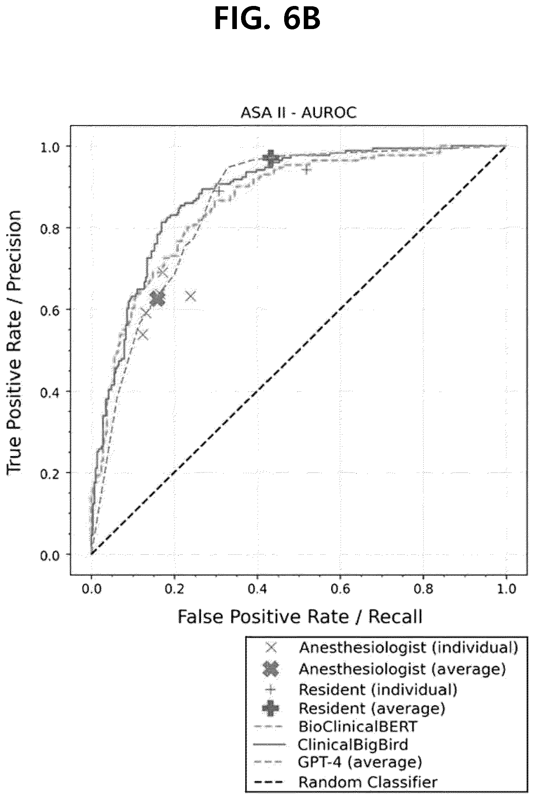

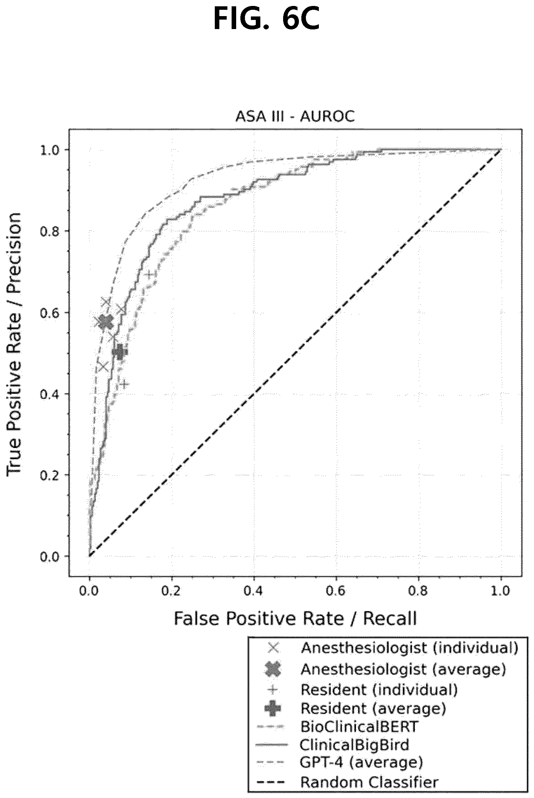

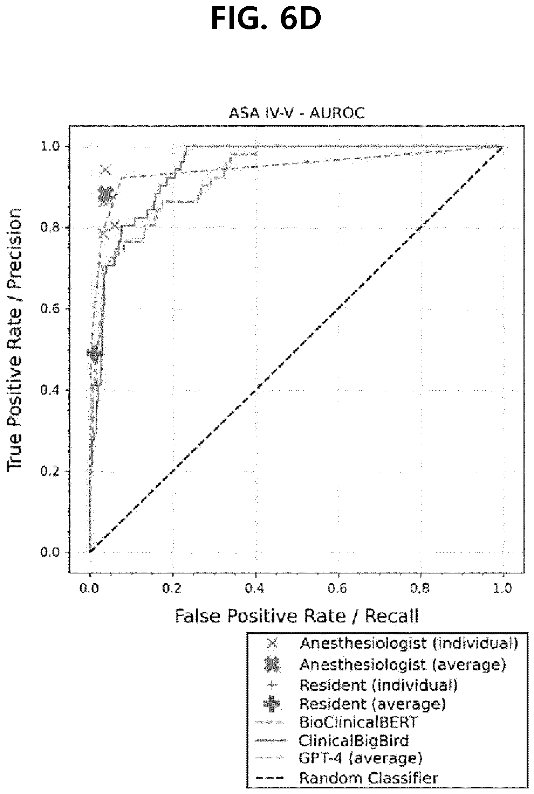

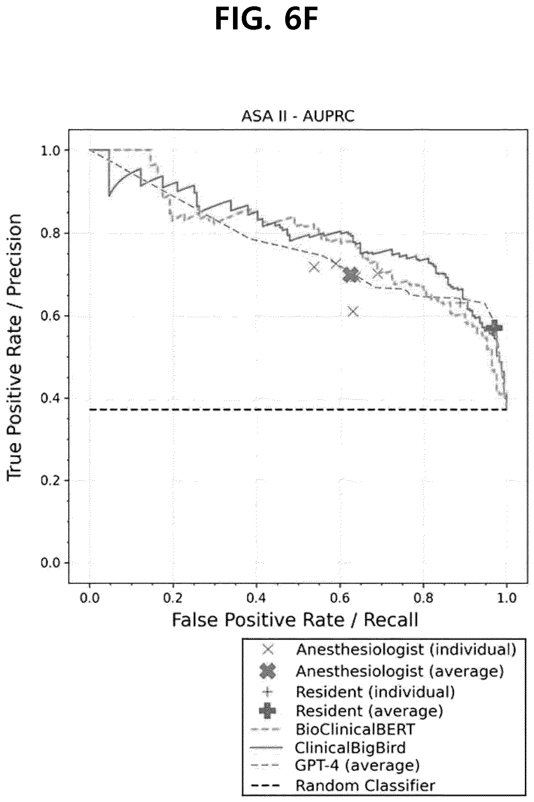

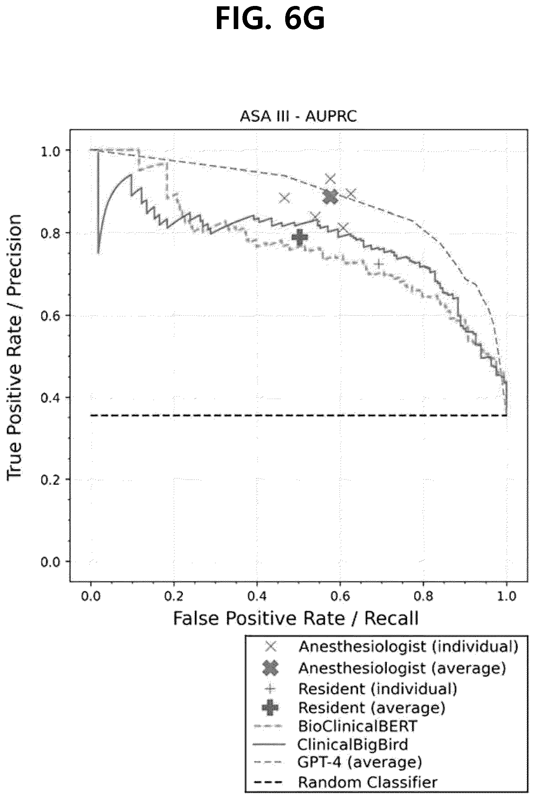

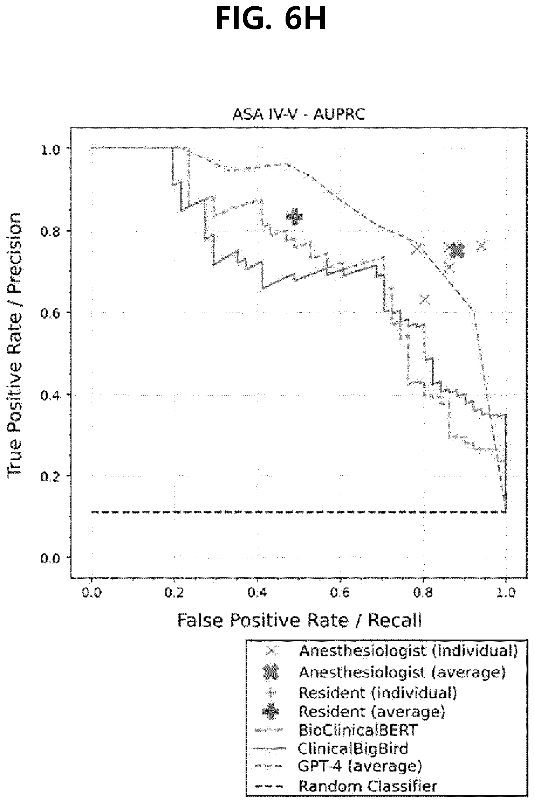

FIG. 6A to FIG. 6H each show a graph comparing the performance of the ClinicalBigBird model, which is the medical classification model of the present application, and other comparison targets (BioClinicalBERT, GPT-4, anesthesiology resident, board-certified anesthesiologist) for each ASA-PS class.

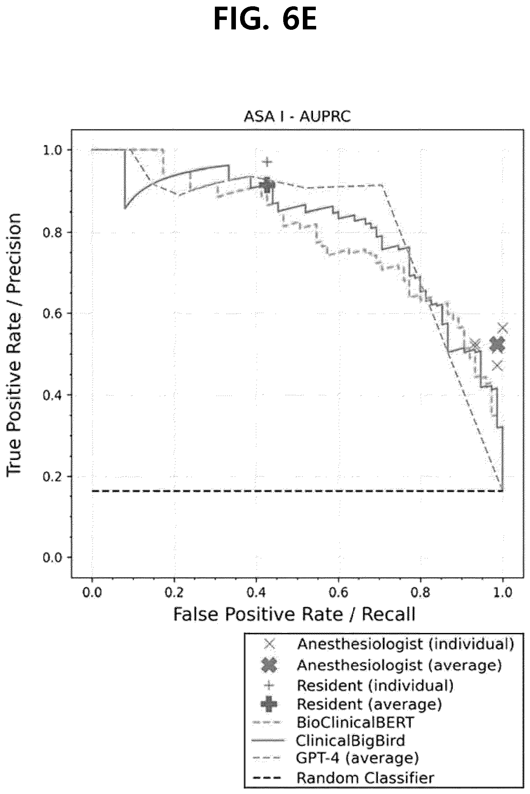

The graphs of FIG. 6A to FIG. 6H each show receiver operating characteristic (ROC) curve for each ASA-PS class (I, II, III, IV-V), and the graphs of FIG. 6E to FIG. 6H show each precision-recall curve. In each graph, the red solid line represents the ClinicalBigBird model, the yellow solid line represents the BioClinicalBERT model, and the purple dotted line represents the average performance of GPT-4. In addition, the 'x' sign of the blue thin line indicates the performance of the individual board-certified anesthesiologist, the 'x' sign of the blue thick line indicates the average performance of the board-certified anesthesiologists, the '+' sign of the green thin line indicates the performances of the individual anesthesiology resident, and the '+' mark of the green thick line indicates the mean performances of the anesthesiology residents.

The following characteristics may be observed from FIG. 6A to FIG. 6H:
- The ClinicalBigBird model achieved an AUROC value of 0.91 or higher across all ASA-PS classes.
- The ClinicalBigBird outperformed the BioClinicalBERT in micro-averaged AUROC (p = 0.010) and was comparable to GPT-4 in macro- and micro-averaged AUROCs.
- In the ASA-PS I and ASA-PS IV-V classes, the models showed particularly high performance, which is interpreted as the characteristic of this extreme class being more clear.
- ASA-PS II and III classes showed relatively low performance because the distinction between these classes is more subtle and requires subjective judgment.
- The ClinicalBigBird model also showed superior or equivalent performance compared to other models in most ASA-PS classes, even in the precision-recall curve.
- Compared to board-certified anesthesiologists, the ClinicalBigBird model showed better performance with specificity 0.901 vs. 0.897, precision 0.732 vs. 0.715, F1-score 0.716 vs. 0.713 (all p<0.01).
- There was a greater difference in performance compared to the anesthesiology residents, with sensitivity 0.723 vs. 0.598, specificity 0.901 vs. 0.868, and F1-score 0.716 vs. 0.633, showing superior performance in all indicators (all p<0.001).

These results show that the ClinicalBigBird model of the present application shows board-certified anesthesiologists level of performance in ASA-PS classification, and may provide stable performance even in the classification of ASA-PS classes II and III, which are particularly important.

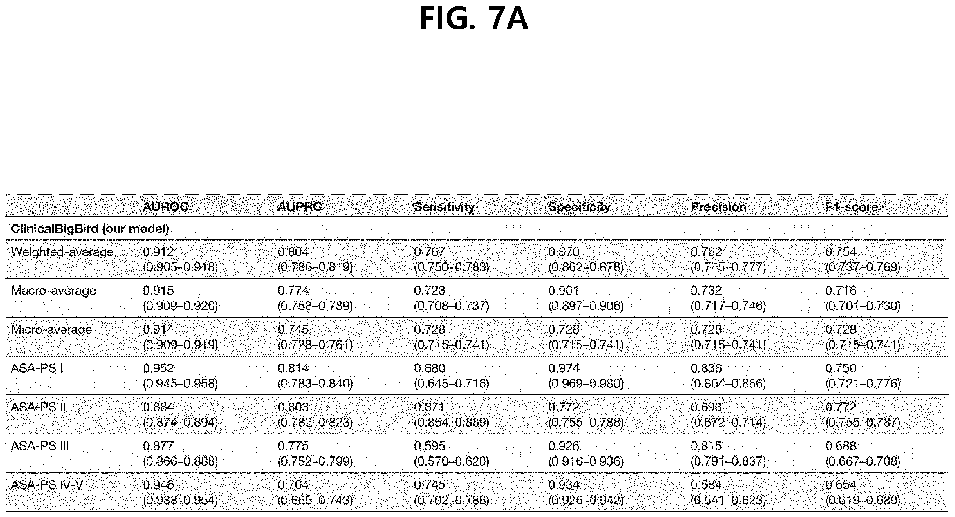

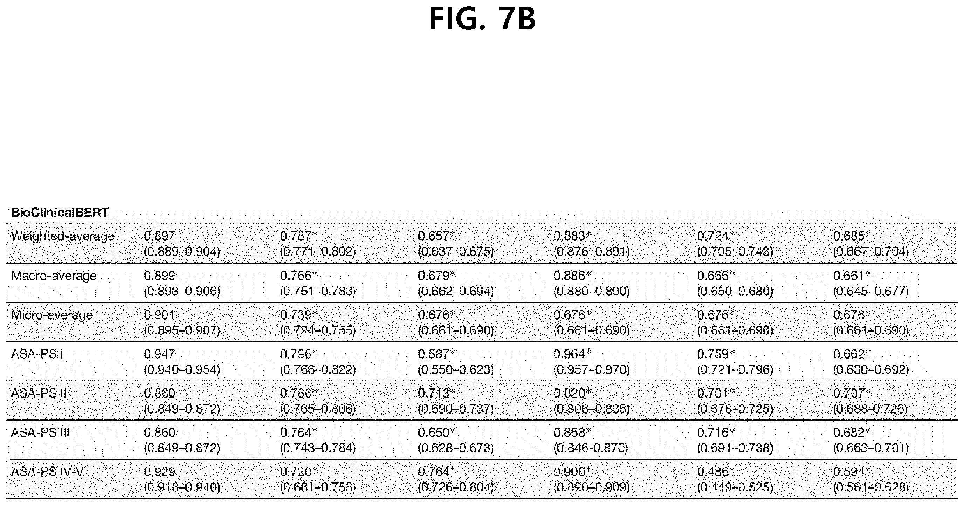

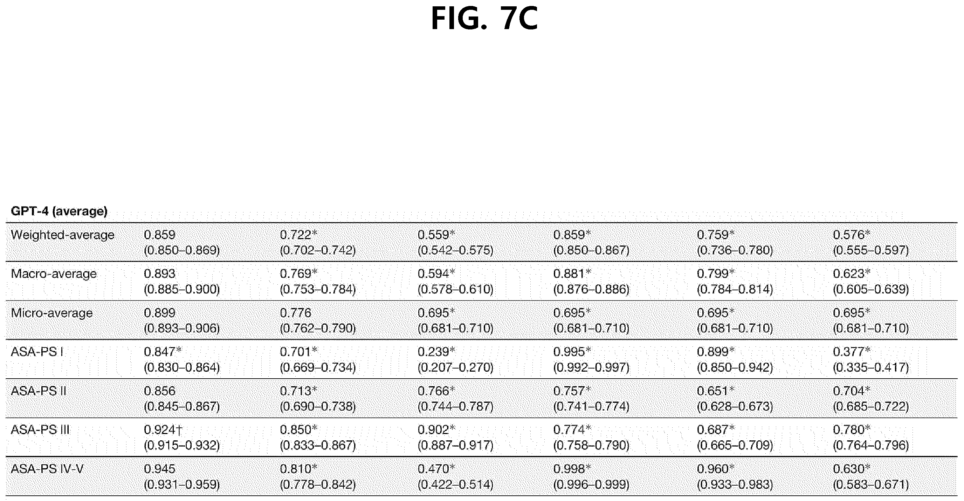

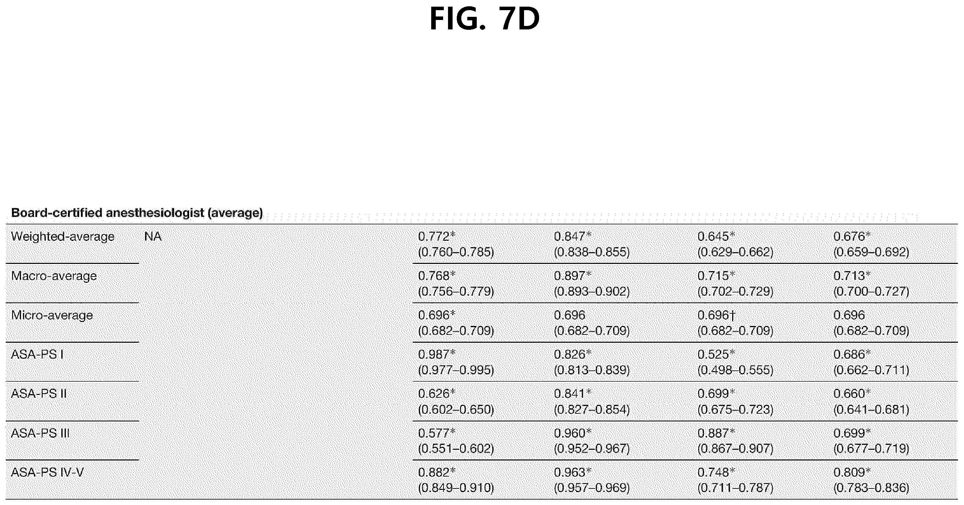

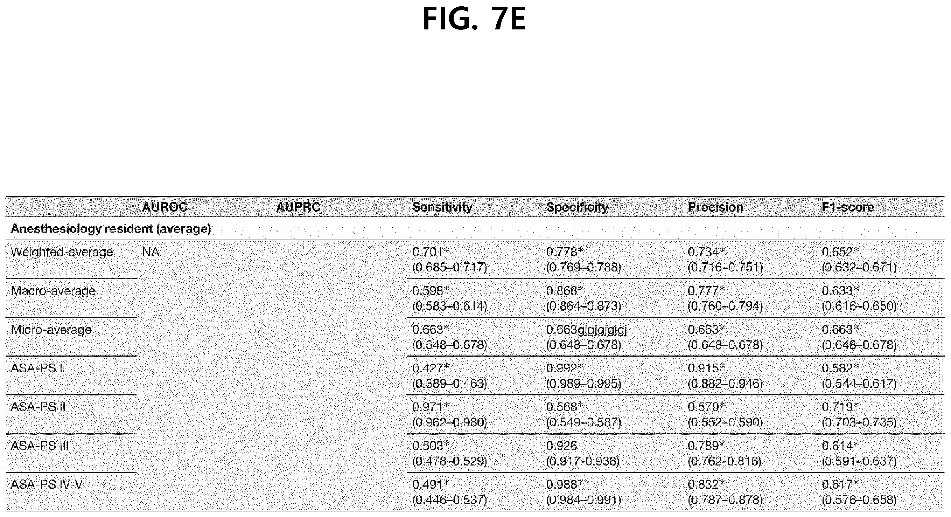

FIG. 7A to FIG. 7E are each a table comparing ASA-PS classification performance of natural language processing models, anesthesiology resident, board-certified anesthesiologist according to an embodiment of the present application.

Referring to FIG. 7A to FIG. 7E, the performance of each model and healthcare professionals was evaluated with six indicators: AUROC, AUPRC, sensitivity, specificity, precision, and F1-score. All values were presented with 95% confidence intervals, indicating statistical significance with the ClinicalBigBird model (* : p < 0.001, † : p < 0.01).

The ClinicalBigBird model of the present application achieved AUROC 0.912(0.905-0.918), AUPRC 0.804(0.786-0.819), sensitivity 0.767(0.750-0.783), specificity 0.870(0.862-0.878), precision 0.762(0.745-0.777), F1-score 0.754(0.737-0.769) on a weighted average basis.

The BioClinicalBERT model showed lower performance than ClinicalBigBird in most indicators, especially with a significantly lower weighted average F1-score 0.685 (0.667-0.704) (p<0.001).

GPT-4 showed superior performance in some indications, but overall showed lower performance than ClinicalBigBird. In particular, the sensitivity was very low at 0.239 (0.207-0.270) in the ASA-PS I class.

The average performance of the board-certified anesthesiologists was sensitivity 0.772 (0.760-0.785), specificity 0.847 (0.838-0.855), precision 0.645 (0.629-0.662), F1-score 0.676 (0.659-0.692), which was generally lower than the ClinicalBigBird model.

The average performance of the anesthesiology residents was sensitivity 0.701 (0.685-0.717), specificity 0.778 (0.769-0.788), precision 0.734 (0.716-0.751), and F1-score 0.652 (0.632-0.671), showing significantly lower performance than the ClinicalBigBird model in all indicators (p<0.001).

Of particular note are the performance differences by ASA-PS class. The ClinicalBigBird model showed the highest AUROC in ASA-PS I and IV-V classes (0.952, 0.946, respectively) and maintained stable performance in ASA- PS II and III classes (0.884, 0.877, respectively).

These results demonstrate that the ClinicalBigBird model of the present application may provide ASA-PS classification performance above the level of a board-certified anesthesiologist, and in particular, shows reliable performance even in the classification of clinically important ASA-PS classes II and III.

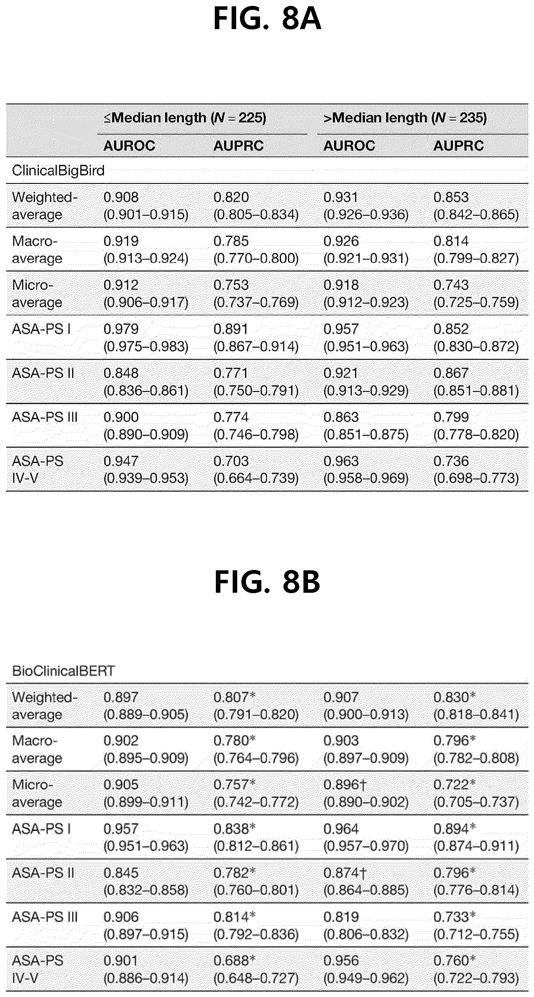

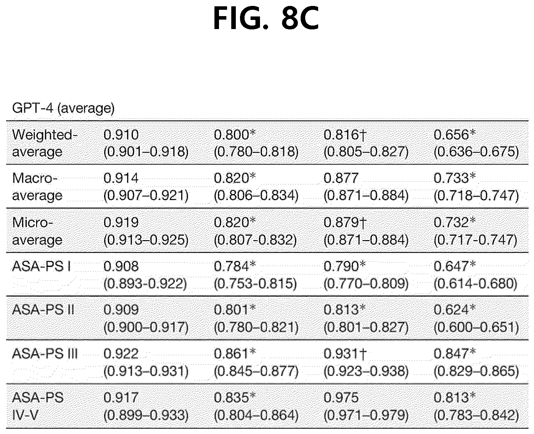

FIG. 8A to FIG. 8C are each a table comparing ASA-PS classification performance of natural language processing models for each subgroup classified based on the length of medical summary information according to an embodiment of the present application.

FIG. 8A to FIG. 8C show the results of comparative analysis of the performance of each model by dividing the dataset into a short group (N=225) and a long group (N=235) based on the median value of the dataset, in order to analyze the effect of the length of the medical summary information on the model performance.

Referring to FIG. 8A to FIG. 8C, the ClinicalBigBird model showed overall superior performance in the long text group. On a weighted average basis, the short text group recorded AUROC 0.908(0.901-0.915) and AUPRC 0.820(0.805-0.834), while the long text group achieved higher performance with AUROC 0.931(0.926-0.936) and AUPRC 0.853(0.842-0.865).

Performance differences with text length were particularly pronounced for ASA-PS II classes. In the long text group, AUROC 0.921(0.913-0.929) and AUPRC 0.867(0.851-0.881) showed significantly higher performance than AUROC 0.848(0.836-0.861) and AUPRC 0.771(0.750-0.791) in the short text group.

The BioClinicalBERT model showed a similar trend, but overall lower performance compared to ClinicalBigBird (p<0.001). ClinicalBigBird significantly outperformed BioClinicalBERT from ASA-PS classes II to IV-V, especially in the long text group.

In the case of GPT-4, the short text group showed relatively stable performance, but the long text group showed a tendency of somewhat decreased performance. In particular, ASA-PS classes I and II showed a large performance difference from ClinicalBigBird.

Overall, the ClinicalBigBird model showed superior performance in medical summary information with longer text lengths, demonstrating that the model has the ability to effectively process text with 4,000 or more tokens. In particular, patients with higher severity tend to have longer medical summary information, and the fact that stable performance may be maintained even in such cases is an important factor in increasing practicality in the clinical field.

All performance indicators were presented with 95% confidence intervals, and statistical significance with the ClinicalBigBird model was indicated as * (p<0.001) or † (p < 0.01).

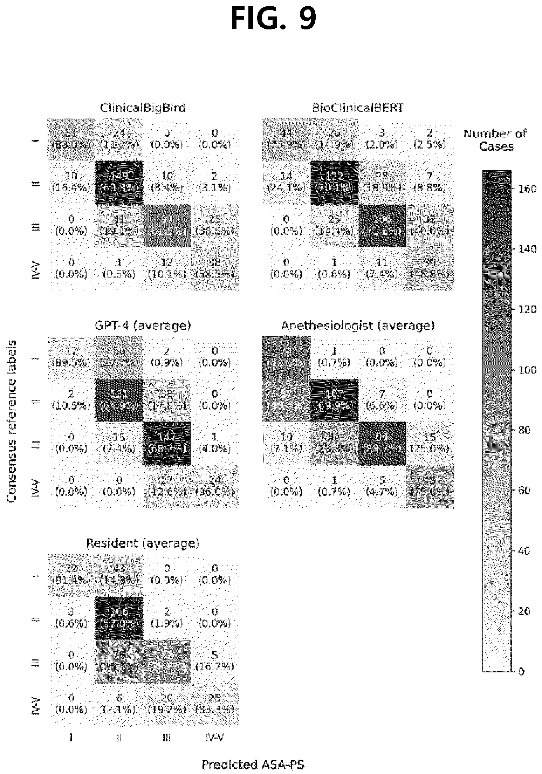

FIG. 9 is a diagram illustrating a confusion matrix for ASA-PS classification of natural language processing models (ClinicalBigBird, BioClinicalBERT, GPT-4) and healthcare professionals (anesthesiology resident, board-certified anesthesiologist) according to an embodiment of the present application.

Referring to FIG. 9, each cell represents the number and percentage of cases of the predicted class (column) relative to the actual ASA-PS class (row). The anesthesiology residents had a strong tendency to classify as ASA-PS II class, and 63.26% of all medical records were classified as ASA-PS II. This shows a tendency to make conservative judgments due to lack of clinical experience.

In the case of board-certified anesthesiologists, there was a tendency to underestimate ASA-PS II as ASA-PS I and ASA-PS III as ASA-PS I or II. Specifically, 33.33% of the ASA-PS II was misclassified as ASA-PS I and 33.13% of the ASA-PS III were misclassified to ASA-PS I or II. It is particularly noteworthy that in the classification of patients with ASA-PS class 3 or higher, the misclassification rate of actual anesthesiologists was 28.2%.

The ClinicalBigBird model significantly improved this classification error. The underestimation rate of ASA-PS II was lowered to 5.85%, and the underestimation ratio of ASA-PS III was lowered to 25.15%, and in particular, the misclassification rate for patients with ASA-PS class 3 or higher was lowered to 19.6%, showing superior performance than the healthcare professionals. However, the increase in the overestimation rate of ASA-PS I from 1.35% to 32.00% was shown as a countervailing effect of this improvement.

GPT-4 showed a marked tendency to overestimate compared to other models and the healthcare professionals group. There was a tendency to misclassify 77.33% of ASA-PS I and 22.22% ASA-PS II as higher classes.

The detailed classification patterns of each model and healthcare professional are as follows:
- ClinicalBigBird: correctly classified 83.6% of ASA-PS I, 69.3% of II, 81.5% of III, 58.5% of IV-V.
- BioClinicalBERT: correctly classified 75.9% of ASA-PS I, 70.1% of II, 71.6% of III, 48.8% of IV-V.
- GPT-4: correctly classified 89.5% of ASA-PS I, 64.9% of II, 68.7% of III, 96.0% of IV-V.
- Anesthesiology residents: correctly classified 52.5% of ASA-PS I, 69.9% of II, 88.7% of III, 75.0% of IV-V.
- Board-certified anesthesiologists: correctly classified 91.4% of ASA-PS I, 57.0% of II, 78.8% of III, 83.3% of IV-V.

These results show that the ClinicalBigBird model shows the most balanced classification performance overall and provides stable performance, especially in distinguishing between clinically important ASA-PS classes II and III. This suggests that the present application may greatly improve the efficiency of the healthcare professionals in anesthesia planning and patient management, and providing a clear description of the prediction of the model may serve as a tool to reduce the workload of the healthcare professionals and effectively support clinical decision-making.

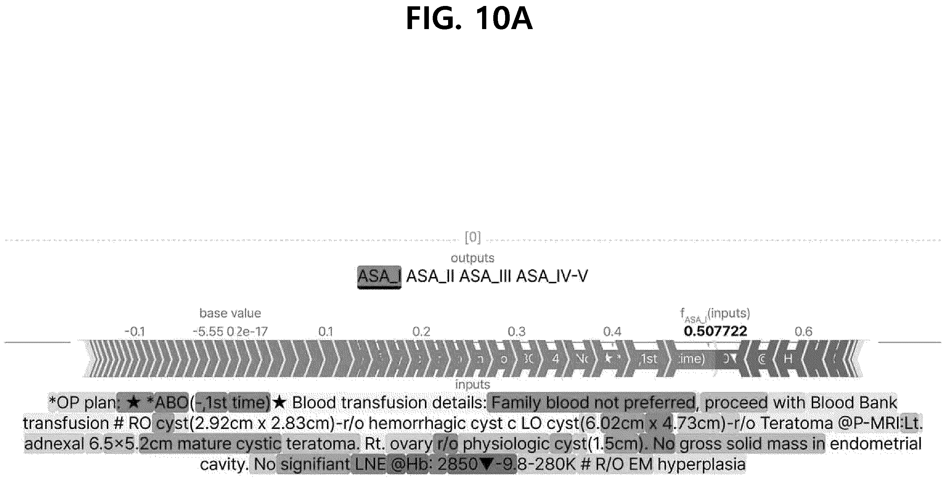

FIG. 10A to FIG. 10D are each a Shapley plot illustrating the impact of input text on each ASA-PS class classification of the ClinicalBigBird model according to an embodiment of the present application.

FIG. 10A to FIG. 10D show results of visualizing the judgment basis of the ASA-PS class predicted by the ClinicalBigBird model utilizing Shapley Additive exPlanations (SHAP). Each Shapley plot represents the contribution of input features (tokens) to the output probability of a specific ASA-PS class.

Referring to FIG. 10A to FIG. 10D, each plot shows the prediction probability for each class from ASA-PS I to IV-V and the contribution of the input features that affected that prediction. The base value of the horizontal axis represents the average model output, and the output value means the final predicted value of the model for the corresponding instance.

The contribution of each token is visualized as arrows, where:
- Token in red: contribute positively towards pushing the model output from the base value to the predicted value, thus acts in the direction of increasing the prediction probability of the corresponding ASA-PS class.
- Token in blue: contribute negatively, thus acts in the direction of lowering the prediction probability of the corresponding ASA-PS class.
- Size of arrow: quantitatively indicate magnitude of the contribution of each token to the final Shapley score.

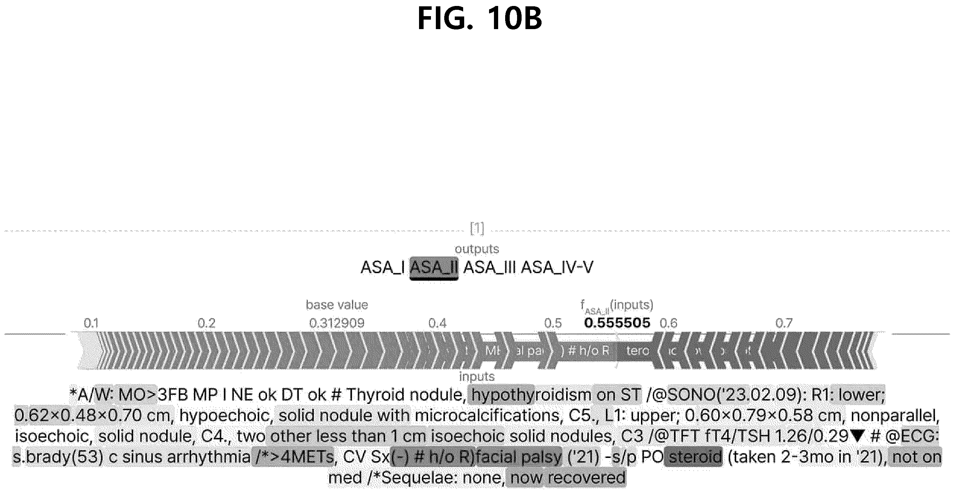

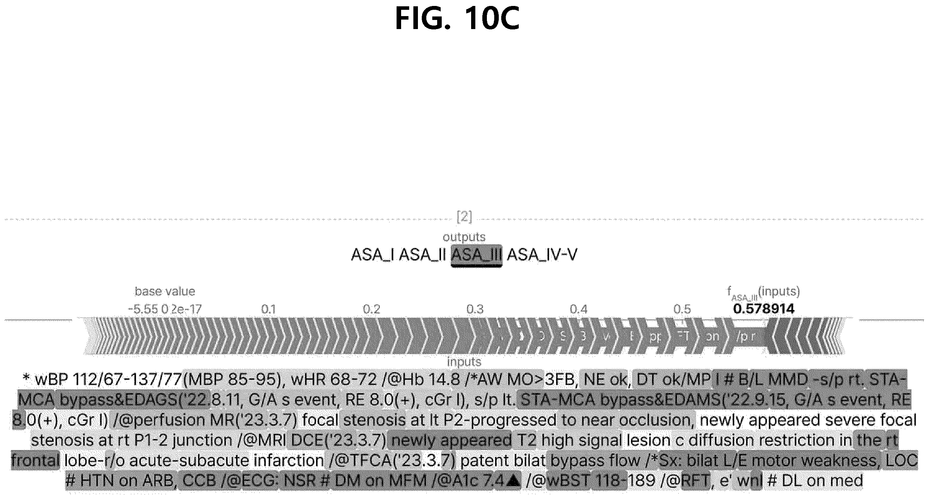

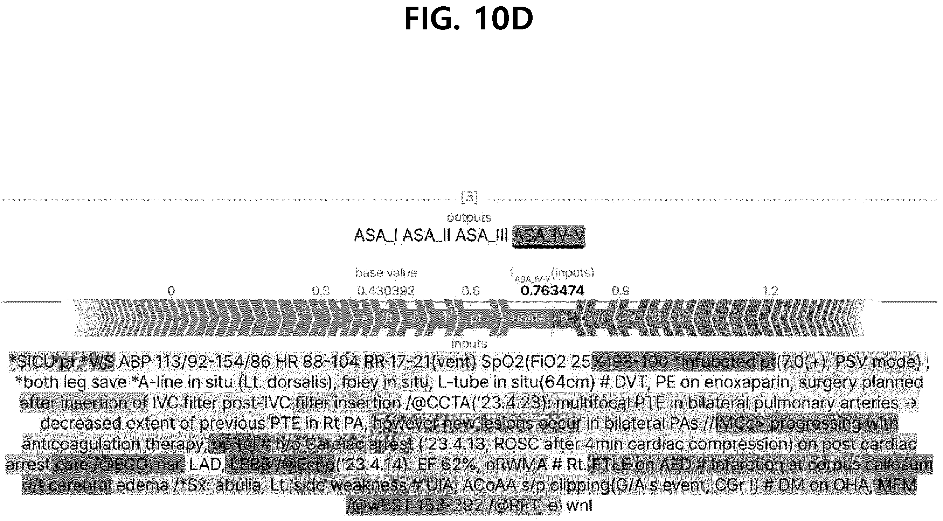

Specific cases are as follows:
- In the case of FIG. 10A, tokens representing normal findings such as 'No significant LNE' in the ASA-PS I class classification contribute to increasing the classification probability.
- In the case of FIG. 10B, tokens representing well-controlled chronic diseases such as 'hypothyroidism on ST' (hypothyroidism) play an important role in the ASA-PS II class classification.
- In the case of FIG. 10C, tokens showing severe systemic diseases such as 'MMD' (Moyamoya disease), 'focal stenosis' (local stenosis), and 'newly appeared T2 high signal lesion' (newly found T2 high signal lesions) in the ASA-PS III class classification show a strong positive contribution.
- In the case of FIG. 10D, tokens representing life-threatening severe conditions such as 'SICU pt' (severe care unit patient), 'Cardiac arrest', and 'Intubated pt' (intubated patient) in the ASA-PS IV-V class classification show a high contribution.

This visualization helps to understand which features (tokens) are driving the model's predictions and their respective contributions to the final Shapley score. The SHAP-based visualization transparently reveal the prediction process of the model, and allows the healthcare professionals to intuitively understand the judgment basis of the model. The clinical characteristics that should be considered important for each class are appropriately reflected, and this shows that the prediction of the model follows a pattern similar to the judgment process of the healthcare professionals.

In addition, the Shapley plot greatly improves the interpretability of the prediction of the model, which provides a basis for the healthcare professionals to understand and trust the judgment basis of the model. In particular, it is possible to quantitatively grasp how much a certain clinical finding or symptom affects a specific ASA-PS class classification, thereby enabling a detailed understanding of the determination process of the model.

Such methods and systems for classification of pre-anesthetic physical status of patients may be implemented in applications or in the form of program instructions that may be performed via various computer components and recorded on a computer-readable recording medium. The computer-readable recording medium may include program instructions, data files, data structures, and the like, alone or in combination.

The program instructions recorded on the computer-readable recording medium may be those specially designed and configured for the present application and those known and usable to those skilled in the computer software field.

Examples of computer-readable recording media include magnetic media such as hard disks, floppy disks, and magnetic tapes, optical recording media such as CD-ROMs, DVDs, magneto-optical media such as floptical disks, and hardware devices specifically configured to store and perform program instructions such as ROMs, RAMs, flash memories, and the like.

Examples of program instructions include machine language codes, such as those made by a compiler, as well as advanced language codes that may be executed by a computer using an interpreter or the like. The hardware device may be configured to operate as one or more software modules to perform processing according to the present application, and vice versa.

Although described above with reference to the embodiments, it will be understood that those skilled in the art may make various modifications and changes to the present application without departing from the spirit and scope of the present application described in the following claims.

## Claims

1. A method S100 for classification of pre-anesthetic physical status of patients, the method comprising:
extracting S110 medical records from an electronic medical record database 210, the medical records comprising at least one or more of surgical information, hospitalization initial diagnosis, nursing initial diagnosis, hospitalization progress, vital signs, test results, and clinical observation records of the patients;
generating S120 medical summary information of patients from the extracted medical record using an artificial intelligence-based natural language processing system;
classifying S140 the pre-anesthetic physical status of the patients using the generated medical summary information as an input of a medical classification model 240; and
visualizing S170 and providing a predictive basis of the medical classification model 240.

2. The method for classification of pre-anesthetic physical status of patients of claim 1, wherein
the medical summary information comprises a text in which Korean and English are used interchangeably,
generating S120, S130 the medical summary information of the patients further comprises performing an accurate translation of the medical terms with reference to a medical terminology dictionary before inputting into the medical classification model 240, and
further comprising correcting a meaning of the medical terms according to the context and translating the text into English.

3. The method for classification of pre-anesthetic physical status of patients of claim 1 or 2, wherein generating S120 the medical summary information of the patients comprises:
analyzing S120 the extracted medical records to automatically identify the primary diagnosis and clinical condition of the patients;
matching S120 the relevant drug use history and the previous surgical history based on the diagnosis content of the patients;
comparing and contrasting S120 a plurality of test results with each other, and classifying the test results by diseases; and
integrating S120 the analysis, matching and classification results into a standardized form of medical summary information.

4. The method for classification of pre-anesthetic physical status of patients of any one of claims 1 to 3, wherein the artificial intelligence-based natural language processing system comprises at least one of a large language model 230 or a multi-agent collaboration network 230.

5. The method for classification of pre-anesthetic physical status of patients of any one of claims 1 to 4, wherein visualizing S170 a predictive basis of the medical classification model 240 comprises
visualizing S170 an impact of each portion of the input text on the prediction results using model explainability methods,
wherein the contribution of key medical terms related to at least one or more of the patient's major disease, surgical history, current condition is highlight, and
separately displaying S170 the contribution of each input text to the prediction result by different visual elements,
wherein a direction and a magnitude of the contribution is distinguishably visualized 250.

6. The method for classification of pre-anesthetic physical status of patients of any one of claims 1 to 5, wherein
(i) the pre-anesthesia physical status of patients comprises an American Society of Anesthesiologists Physical Status (ASA-PS) class, optionally wherein the ASA-PS class is classified into one of classes I, II, III, IV-V, and the method further comprises determining S150, S160 whether to apply additional anesthesia fees for the patients classified as ASA-PS class III or higher; and/or
(ii) for learning the medical classification model 240, the method comprises:
a training step 311 using a first dataset;
a tuning step 312 using a second dataset; and
a testing step 313 using a third dataset,
wherein the second dataset and the third dataset comprise samples selected through a sampling method that takes into account each pre-anesthesia physical status class, and are configured such that no data from the same patient is overlapped between the second dataset and the third dataset.

7. The method for classification of pre-anesthetic physical status of patients of claim 6, wherein, when feature (ii) is present,
a plurality of board-certified anesthesiologists independently perform an evaluation on the second dataset and the third dataset, and
when the evaluation result is inconsistent, a reference label is generated by final agreement through additional board-certified anesthesiologist consultation,
wherein the evaluation is performed after removing a part explicitly mentioned in the ASA-PS classification information from the medical summary information.

8. The method for classification of pre-anesthetic physical status of patients of any one of claims 1 to 7, wherein
(i) a plurality of predictions S140 are performed to estimate an uncertainty S180 for the prediction of the medical classification model 240, wherein the uncertainty is estimated by distinguishing between the uncertainty due to variability inherent in the input data and the uncertainty due to the model parameters; and/or
(ii) the method further comprises classifying S190 the input text into subgroups according to the length of the text, and evaluating S190 the performance of the medical classification model 240 for each subgroup.

9. A computer-readable recording medium having recorded thereon a program for executing the method of any one of claims 1 to 8 in a computer.

10. A system for classification of pre-anesthetic physical status of patients, the system comprising:
an electronic medical record database 210;
an extracting unit 220 configured to extract medical records from the electronic medical record database 210, the medical records comprising at least one or more of surgical information, hospitalization initial diagnosis, nursing initial diagnosis, hospitalization progress, vital signs, test results, and clinical observation records of the patients;
a summary generation unit 230 configured to generate medical summary information of patients from the extracted medical record using an artificial intelligence-based natural language processing system;
a medical classification model unit 240 configured to classify the pre-anesthetic physical status of the patients using the generated medical summary information as an input of a medical classification model 245; and
a visualization unit 250 configured to visualize and provide a predictive basis of the medical classification model 245.

11. The system for classification of pre-anesthetic physical status of patients of claim 10, wherein
the medical summary information comprises a text in which Korean and English are used interchangeably,
the summary generation unit 230 performs an accurate translation of the medical terms with reference to a medical terminology dictionary, and corrects a meaning of the medical terms according to a context.

12. The system for classification of pre-anesthetic physical status of patients of claim 10 or 11, wherein the summary generation unit 230 is configured to perform:
a function of analyzing the extracted medical records to automatically identify the primary diagnosis and clinical condition of the patients;
a function of matching the relevant drug use history and the previous surgical history based on the diagnosis content of the patients;
a function of comparing and contrasting a plurality of test results with each other, and classifying the test results by diseases; and
a function of integrating the analysis, matching and classification results into a standardized form of medical summary information.

13. The system for classification of pre-anesthetic physical status of patients of any one of claims 10 to 12, wherein
(i) the artificial intelligence-based natural language processing system comprises at least one of a large language model or a multi-agent collaboration network; and/or
(ii) the visualization unit 250 visualizes an impact of each portion of the input text on the prediction results using model explainability methods, wherein the contribution of key medical terms related to at least one or more of the patient's major disease, surgical history, current condition is highlight, and separately display the contribution of each input text to the prediction result by different visual elements, wherein a direction and a magnitude of the contribution is distinguishably visualized.

14. The system for classification of pre-anesthetic physical status of patients of any one of claims 10 to 13, wherein
(i) the pre-anesthesia physical status of patients comprises an American Society of Anesthesiologists Physical Status (ASA-PS) class, and further comprising an anesthesia fee calculation unit 260 configured to determine whether to apply additional anesthesia fees for patients classified as ASA-PS class III or higher; and/or
(ii) the medical classification model unit 240 comprises a transformer-based model 245 capable of processing a long medical text.

15. The system for classification of pre-anesthetic physical status of patients of any one of claims 10 to 14, wherein
the learning data of the medical classification model 240 comprises samples selected through a sampling method that takes into account each pre-anesthesia physical status class, and
the samples comprise reference labels generated through evaluation by a plurality of board-certified anesthesiologists.
